# EUROPÄISCHE PATENTANMELDUNG

(11) **EP 3 682 795 A1**
(43) Veröffentlichungstag der Anmeldung: **22.07.2020**
(21) Anmeldenummer: 19211859.4
(22) Anmeldetag: 10.01.2013
(51) Int. Cl.: A61B 3/16, A61F 2/16

(54) **IMPLANTATVORRICHTUNG, SENSORMODUL, EINWEGINJEKTOR MIT EINER IMPLANTATVORRICHTUNG UND VERFAHREN ZUM HERSTELLEN EINER IMPLANTATVORRICHTUNG**

(30) Priorität: 17.01.2012 DE 102012200574
(62) Teilanmeldung aus: 13700542.7
(71) Anmelder: Implandata Ophthalmic Products GmbH, 30159 Hannover (DE)
(72) Erfinder: Scholten, Dick, 70188 Stuttgart (DE)
(74) Vertreter: Knoop, Philipp

(57) **Zusammenfassung**

Die Erfindung betrifft eine Implantatvorrichtung mit einem Sensormodul, welches ein oder mehrere erste Befestigungseinrichtungen aufweist, einem Implantatmodul, welches ein oder mehrere zweite Befestigungseinrichtungen aufweist. und mit einem Verbindungsmodul, mittels welchem das Sensormodul mit dem Implantatmodul über die eine oder die ersten Befestigungseinrichtungen und über die eine oder die zweiten Befestigungseinrichtungen verbunden ist, und mit einer Faltachse, entlang welcher die Implantatvorrichtung zusammenklappbar Ist.

## Beschreibung

Die Erfindung betrifft eine Implantatvorrichtung, ein Sensormodul, einen Einweginjektor mit einer Implantatvorrichtung und ein Verfahren zum Herstellen einer Implantatvorrichtung.

### Stand der Technik

Glaukom ist eine der häufigsten Erkrankungen des Sehnervs. Charakteristisch ist ein kontinuierlicher Verlust von Nervenfasern, was zu Gesichtsfeldausfällen und im Extremfall zu einer Erblindung des Auges führen kann. Als wichtigster Risikofaktor wird ein zu hoher Augeninnendruck angesehen.

Für eine optimale Therapie ist es notwendig den Krankheitsverlauf und die Risikofaktoren wie etwa den Augeninnendruck genau zu verfolgen. Als Augeninnendruck bezeichnet man den physikalischen Druck, der auf der Augeninnenwand lastet. Er bewirkt eine konstant glatte Wölbung der Hornhautoberfläche und einen gleich bleibenden Abstand zwischen Hornhaut, Linse und Netzhaut des Auges, sowie eine gleichmäßige Ausrichtung der Fotorezeptoren auf der Netzhaut. Zudem hält der Augeninnendruck die stabile Kugelform des Augapfels aufrecht. Für eine Bestimmung des Augeninnendrucks gibt es entsprechende Messgeräte in den Arztpraxen.

Wenn der Augeninnendruck bestimmte Grenzwerte für bestimmte Zeitspannen überschreitet, kann der intrazelluläre Transport in den Nervenfasern derart beeinträchtigt werden, dass die Nervenfasern absterben.

Da vor allem der Augeninnendruck sehr stark schwanken kann, ist eine häufige Messung für eine genaue Diagnose notwendig. Die direkteste Messung wäre eine Messung direkt im Auge. Idealerweise möchte man eine Implantation vermeiden. Allerdings werden jährlich sehr viele Intraokularlinsen implantiert, allein im Jahr 2007 in Deutschland 600.000 Stück.

Die WO 2001 021 063 A1 beschreibt eine Vorrichtung zum Messen von physikalischen Größen im Auge, insbesondere des Augeninnendrucks mit einem Telemetriesystem mit einem in der Intraokularlinse integrierten Drucksensor. Bei der Herstellung der dort beschrieben Vorrichtung wird in einem Silikongussform-Prozess gleichzeitig der Drucksensor verkapselt und die Intraokularlinse geformt. Dabei ist ein Schnitt von meist mehr als 3 mm im Auge notwendig, um eine solche Intraokularlinse zu implantieren. Ferner ist prozessbedingt nur Silikon als Linsenmaterial verwendbar, weil andere Materialien nicht mit einem Silikongussform-Prozess anwendbar sind. Durch die feste mechanische Verbindung zwischen Drucksensormodul und Intraokularlinse ist eine Verbiegung des Drucksensormoduls möglich, durch die die optische Abbildung der Intraokularlinse verzerrt wird.

### Offenbarung der Erfindung

Die vorliegende Erfindung schafft gemäß einem Aspekt eine Implantatvorrichtung mit einem Sensormodul, welches ein oder mehrere erste Befestigungseinrichtungen aufweist, mit einem Implantatmodul, welches ein oder mehrere zweite Befestigungseinrichtungen aufweist, und mit einem Verbindungsmodul, mittels welchem das Sensormodul mit dem Implantatmodul über die eine oder die ersten Befestigungseinrichtungen und über die eine oder die zweiten Befestigungseinrichtungen verbunden ist, und mit einer Faltachse, entlang welcher die Implantatvorrichtung zusammenklappbar ist.

Gemäß einem weiteren Aspekt schafft die vorliegende Erfindung ein Sensormodul mit: einer oder mehreren ersten Befestigungseinrichtungen, welche zum Fixieren eines Verbindungsmoduls ausgelegt sind; und mindestens einer Faltachse, entlang welcher das Sensormodul zusammenklappbar ist.

Gemäß einem weiteren Aspekt schafft die vorliegende Erfindung einen Einweginjektor mit einer Implantatvorrichtung nach Patentanspruch 14.

Gemäß einem weiteren Aspekt schafft die vorliegende Erfindung ein Verfahren zum Herstellen einer Implantatvorrichtung mit den Merkmalen des Patentanspruchs 15.

### Vorteile der Erfindung

Eine Idee der vorliegenden Erfindung ist es, eine faltbare oder zusammenklappbare Verbindung zwischen Sensormodul und Intraokularlinse bereitzustellen.

Insbesondere soll die Verbindungen zwischen Sensormodul und Intraokularlinse flexibel ausgestaltet sein. Dadurch sind Verlegungen des Sensormoduls weniger kritisch für die Intraokularlinse. Vorteilhaft erlaubt die flexible Verbindung auch, die Intraokularlinse und das Sensormodul sukzessiv zu implantieren. Somit ist eine geringere Schnittgröße im Auge erforderlich, besonders bei einer Verwendung von faltbaren Sensormodulen. Ferner ergibt sich mit einer Verwendung einer nicht-monolithischen flexiblen Verbindung die Möglichkeit, andere Linsenmaterialien, wie Polymethylmethacrylat, Kurzzeichen PMMA, umgangssprachlich Acrylglas oder Plexiglas, oder Hydrogele zu verwenden, d.h. Wasser enthaltende, aber wasserunlösliche Polymere, deren Moleküle chemisch durch kovalente oder ionische Bindungen oder physikalisch durch ein Verschlaufen der Polymerketten, zu einem dreidimensionalen Netzwerk verknüpft sind.

Für eine optimale optische Abbildung muss die Intraokularlinse an die richtige Stelle im Auge positioniert und fixiert werden. Die Intraokularlinse kann dann als Rahmen fungieren, an dem das Sensormodul entfaltet und positioniert werden kann.

Gemäß einer Ausführungsform der Erfindung ist das Implantatmodul als eine Intraokulaorlinse ausgebildet.

Gemäß einer weiteren Ausführungsform der Erfindung ist das Implantatmodul als ein Kapselspannring ausgebildet.

Gemäß einer weiteren Ausführungsform der Erfindung ist das Sensormodul als ein Drucksensormodul ausgebildet.

Gemäß einer weiteren Ausführungsform der Erfindung sind die eine oder die mehreren ersten Befestigungseinrichtungen des Sensormoduls als Ösen ausgebildet.

Gemäß einer weiteren Ausführungsform der Erfindung sind die eine oder die mehreren zweiten Befestigungseinrichtungen des Implantatmoduls als Ösen ausgebildet.

Gemäß einer weiteren Ausführungsform der Erfindung ist die Implantatvorrichtung zur Verwendung in einem Injektor oder einem Injektorset vorgesehen, mit welchem die Implantatvorrichtung zu implantieren ist.

Die beschriebenen Ausgestaltungen und Weiterbildungen lassen sich, sofern sinnvoll, beliebig miteinander kombinieren.

Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.

Es zeigen:
- Fig. 1: eine schematische Darstellung eines Sensormoduls gemäß einer Ausführungsform der Erfindung;
- Fig. 2: eine schematische Darstellung einer Implantatvorrichtung vor einem Durchführen durch eine Operationsöffnung gemäß einer weiteren Ausführungsform der Erfindung;
- Fig. 3: eine schematische Darstellung einer Implantatvorrichtung nach einem Durchführen durch eine Operationsöffnung gemäß einer weiteren Ausführungsform der Erfindung;
- Fig. 4-7: jeweils eine schematische Darstellung einer Implantatvorrichtung gemäß einer weiteren Ausführungsform der Erfindung; und
- Fig. 8: eine schematische Darstellung eines Flussdiagramms des Verfahrens zum Herstellen einer Implantatvorrichtung gemäß einer weiteren Ausführungsform der Erfindung.

In den Figuren der Zeichnung bezeichnen gleiche Bezugszeichen gleiche oder funktionsgleiche Elemente, Bauteile, Komponenten oder Verfahrensschritte, soweit nichts Gegenteiliges angegeben ist.

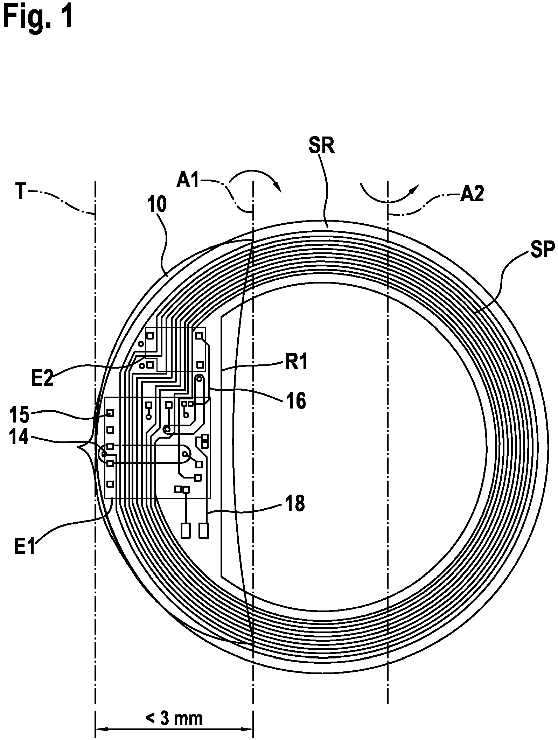

Die Figur 1 zeigt eine schematische Darstellung eines Sensormoduls gemäß einer Ausführungsform der Erfindung.

Eine Sensormodul 10 weist eine oder mehrere Faltachsen A1, A2 auf, entlang welcher das Sensormodul zusammenklappbar ist. Beispielsweise sind die Faltachsen A1, A2 parallel zu einer Tangente T des Sensormoduls 10 ausgebildet.

Das Sensormodul 10 ist auf einem faltbaren Träger SR integriert und umfasst ferner eine in den faltbaren Träger SR integrierte Spule SP, welche beispielsweise in einer ebenen Fläche in Form mehrerer nebeneinander liegender Spulenwindungen planar ausgebildet ist.

Die Elektronik des Sensormoduls 10 ist beispielsweise in Schaltungseinrichtungen E1, E2 integriert, welche flexible Leiterbahnen 14, 15, 16, 18 und weitere Bauelemente aufweisen. Die Elektronik kann in einem eigenen Elektronikträger R1 integriert sein.

Das Sensormodul 10 ist beispielsweise mit Parylen oder einem sonstigen inerten polymeren Beschichtungsmaterial beschichtet.

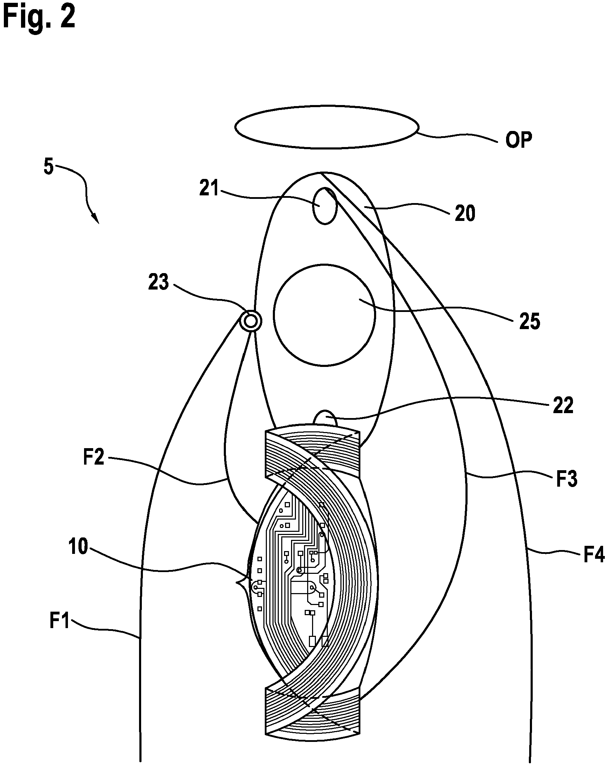

Die Figur 2 zeigt eine schematische Darstellung einer Implantatvorrichtung vor einem Durchführen durch eine Operationsöffnung OP gemäß einer weiteren Ausführungsform der Erfindung.

Eine Implantatvorrichtung 5 umfasst ein Sensormodul 10 und ein Implantatmodul 20. Das Implantatmodul 20 weist ein oder mehrere zweite Befestigungselemente 21, 22, 23 auf.

Beispielsweise sind die Befestigungselemente 21, 22, 23 zur Aufnahme von Fäden F1-F4 ausgebildet. Vorteilhaft sind die Befestigungselemente 21, 22, 23 als Ösen oder als ein sonstiges Befestigungselement aus einem biokompatiblen Kunststoff oder Metall gebildeter Ring ausgebildet.

Mittels der Fäden F1-F4 lässt sich die Implantatvorrichtung 5 nach dem Durchschieben durch die Operationsöffnung OP entfalten. Somit kann die Implantatvorrichtung 5 an ihrer endgültigen Position im Köperinneren durch eine über die Fäden F1-F4 angewandte Zugkraft wieder aufgeklappt werden.

Hierbei wird beispielsweise das Implantatmodul mit einer Pinzette festgehalten und gleichzeitig an den Faden F1 und/oder F4 eine Zugkraft ausgeübt.

Das Implantatmodul 20 ist beispielsweise mit Parylen oder einem sonstigen inerten polymeren Beschichtungsmaterial beschichtet.

Das Implantatmodul 20 ist beispielsweise als eine Intraokulaorlinse oder als eine sonstige im Auge zu implantierende künstliche Linse ausgebildet und weist eine Linse 25 auf. Die Spule SP und der zugehörige Träger SR des Sensormoduls 10 der Implantatvorrichtung 5 sind dabei konzentrisch zu der Linse 25 des Implantatmoduls 20 angeordnet.

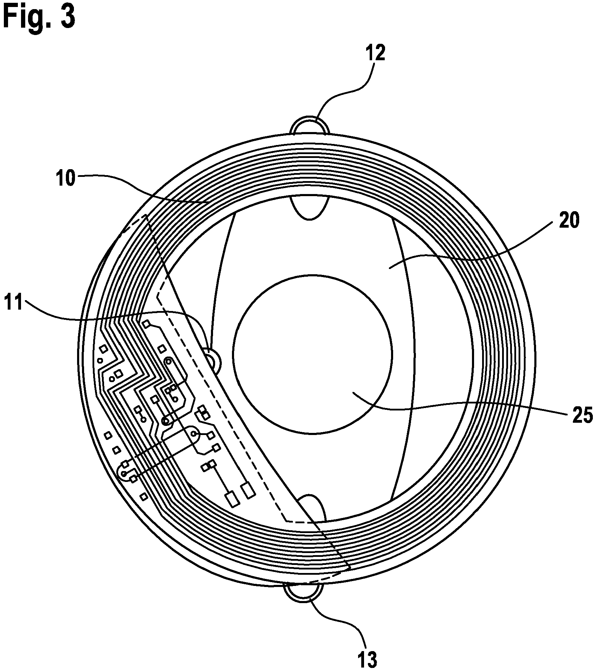

Die Figur 3 zeigt eine schematische Darstellung einer Implantatvorrichtung nach einem Durchführen durch eine Operationsöffnung gemäß einer weiteren Ausführungsform der Erfindung.

Die Implantatvorrichtung 5 umfasst das Sensormodul 10 und das Implantatmodul 20. Das Sensormodul 10 weist eine oder mehrere erste Befestigungseinrichtungen 11, 12, 13 auf, welche zum Fixieren eines Verbindungsmoduls 30 ausgelegt sind.

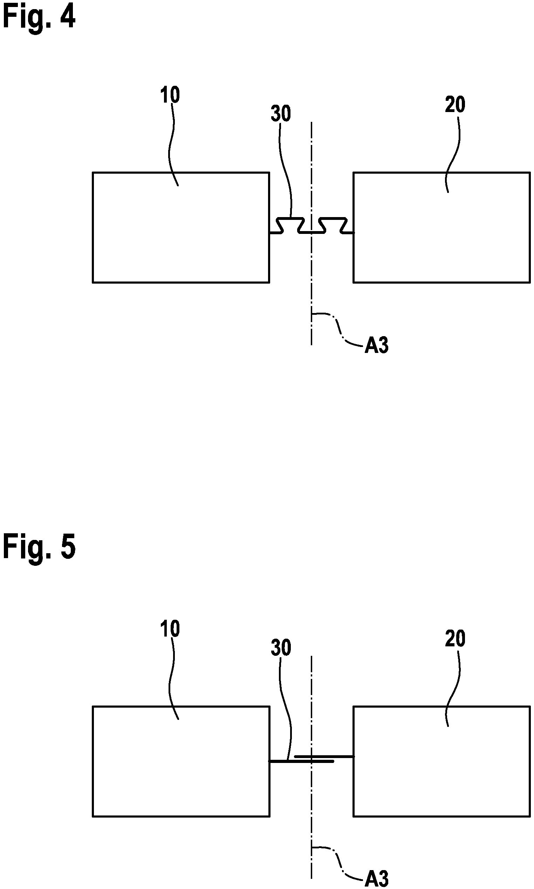

Die Figuren 4 bis 7 zeigen jeweils eine schematische Darstellung einer Implantatvorrichtung gemäß einer weiteren Ausführungsform der Erfindung.

Die weiteren in der Figur 3 verwendeten Bezugszeichen sind bereits in der zu der Figur 2 zugehörigen Figurenbeschreibung beschrieben und werden daher nicht weiter erläutert.

In der Figur 4 ist eine Implantatvorrichtung 5 mit einem flexiblen Verbindungsmodul 30 dargestellt. Das in der Figur 4 gezeigte Verbindungsmodul 30 ist beispielsweise aus einem bieg- oder knickbaren Material ausgebildet. Durch die Verwendung des flexiblen Verbindungsmoduls 30 weist die Implantatvorrichtung 5 eine Faltachse A3 auf, entlang welcher die Implantatvorrichtung 5 zusammenklappbar ist.

Die weiteren in der Figur 4 verwendeten Bezugszeichen sind bereits in der zu der Figur 2 zugehörigen Figurenbeschreibung beschrieben und werden daher nicht weiter erläutert.

Das in der Figur 5 gezeigte Verbindungsmodul 30 ist beispielsweise als ein flexibles Scharnierteil ausgebildet ist. Durch das Scharnierteil weist die Implantatvorrichtung 5 eine Faltachse A3 auf, entlang welcher die Implantatvorrichtung 5 zusammenklappbar ist.

Die weiteren in der Figur 5 verwendeten Bezugszeichen sind bereits in der zu der Figur 2 zugehörigen Figurenbeschreibung beschrieben und werden daher nicht weiter erläutert.

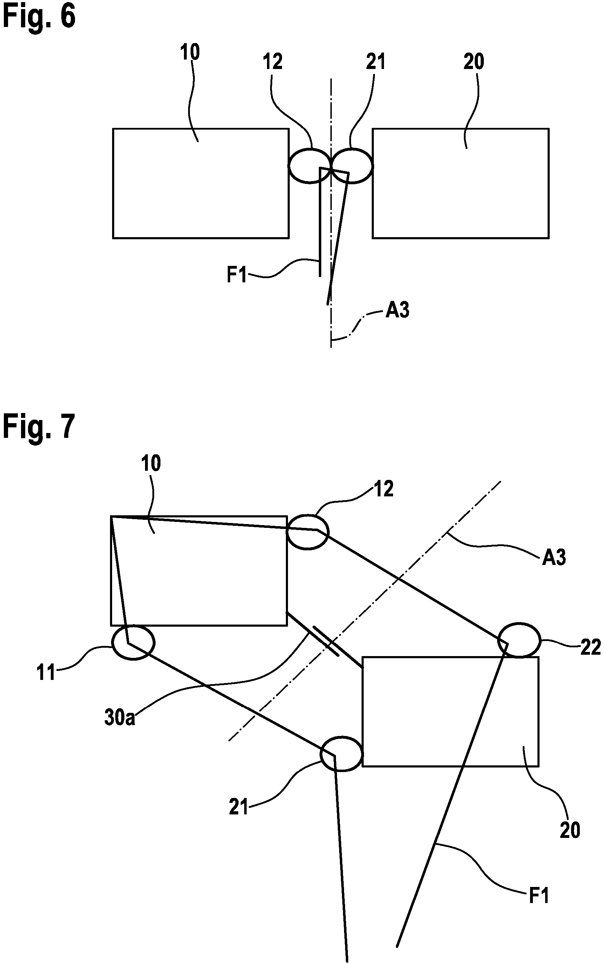

Die in der Figur 6 gezeigte Implantatvorrichtung 5 weist ein Verbindungsmodul 30 auf, welches beispielsweise durch einen Faden F1 und eine erste Befestigungseinrichtung 11 des Sensormoduls 10 und eine zweite Befestigungseinrichtung 21 des Implantatmoduls 20 ausgebildet wird.

Die weiteren in der Figur 6 verwendeten Bezugszeichen sind bereits in der zu der Figur 2 zugehörigen Figurenbeschreibung beschrieben und werden daher nicht weiter erläutert.

Das in der Figur 7 gezeigte Verbindungsmodul 30 wird beispielsweise zum einen durch ein Scharnierteil 30a und zum anderen durch einen Faden F1 und zwei erste Befestigungseinrichtungen 11,12 des Sensormoduls 10 und zwei zweite Befestigungseinrichtungen 21,22 des Implantatmoduls 20 ausgebildet.

Die weiteren in der Figur 7 verwendeten Bezugszeichen sind bereits in der zu der Figur 2 zugehörigen Figurenbeschreibung beschrieben und werden daher nicht weiter erläutert.

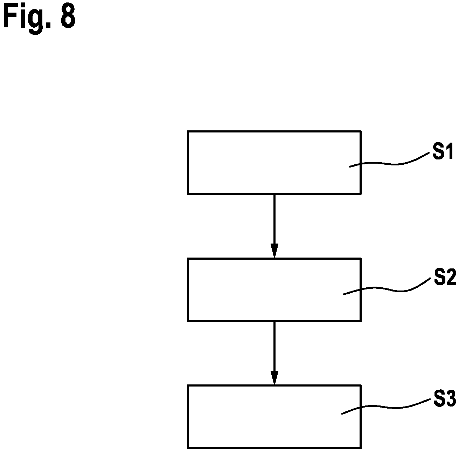

Die Figur 8 zeigt eine schematische Darstellung eines Flussdiagramms des Verfahrens zum Herstellen einer Implantatvorrichtung gemäß einer weiteren Ausführungsform der Erfindung.

Das Verfahren zum Herstellen einer Implantatvorrichtung umfasst die folgenden Verfahrensschritte:
Als ein erster Verfahrensschritt erfolgt ein Bereitstellen S1 eines Sensormoduls 10, eines Implantatmoduls 20 und eines Verbindungsmoduls 30.

Als ein zweiter Verfahrensschritt erfolgt ein Montieren S2 des Sensormoduls 10, des Implantatmoduls 20 und des Verbindungsmoduls 30 zur Implantatvorrichtung 5, wobei beispielsweise die Fäden F1-F4 durch die als Ösen ausgebildeten ersten Befestigungseinrichtungen 11-13 oder durch die als Ösen ausgebildeten zweiten Befestigungseinrichtungen 21-23 des Sensormoduls 10 bzw. des Implantatmoduls 20 hindurchgezogen werden.

Als ein dritter Verfahrensschritt erfolgt ein Falten S3 der montierten Implantatvorichtung 5 entlang mindestens einer Faltachse A3 der Implantatvorichtung 5 zum Zusammenklappen der Implantatvorrichtung 5.

## Patentansprüche

1. Sensormodul mit:
- einer oder mehreren ersten Befestigungseinrichtungen (11-13), welche zum Fixieren eines Verbindungsmoduls (30) ausgelegt sind; und
- mindestens einer Faltachse (A1, A2), entlang welcher das Sensormodul zusammenklappbar ist.

2. Sensormodul nach Anspruch 1, wobei das Sensormodul ferner eine Aktuatorvorrichtung zur automatisierten Medikamentenabgabe aufweist.
